(19) Europäisches Patentamt
European Patent Office
Office européen des brevets

(11) EP 1 838 758 B1

# (12) EUROPEAN PATENT SPECIFICATION

(45) Date of publication and mention of the grant of the patent:
**12.04.2017 Bulletin 2017/15**

(21) Application number: **05854540.1**

(22) Date of filing: **16.12.2005**

(51) Int Cl.:
***C08G 77/18*** *(2006.01)* ***C08G 77/20*** *(2006.01)*
***B29D 11/00*** *(2006.01)*

(86) International application number:
**PCT/US2005/045847**

(87) International publication number:
**WO 2006/068986 (29.06.2006 Gazette 2006/26)**

## (54) POLYSILOXANES, METHOD OF SYNTHESIS AND OPHTHALMIC COMPOSITIONS

POLYSILOXANE, SYNTHESEVERFAHREN UND OPHTHALMISCHE ZUSAMMENSETZUNGEN

POLYSILOXANES, MÉTHODE DE SYNTHÈSE ET PRÉPARATIONS OPHTHALMIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.12.2004 US 637861 P**
**20.12.2004 SE 0403093**

(43) Date of publication of application:
**03.10.2007 Bulletin 2007/40**

(73) Proprietor: **AMO Groningen B.V.**
**9728 NX Groningen (NL)**

(72) Inventors:
- **LIU, Yan**
**SE-281 33 Hassleholm (SE)**
- **HILBORN, Jons Gunnar**
**SE-193 30 Sigtuna (SE)**
- **HAITJEMA, Hendrik Jan**
**NL-9321 Peize (NL)**

(74) Representative: **Bergenstråhle Group AB**
**P.O. Box 17704**
**118 93 Stockholm (SE)**

(56) References cited:
**EP-A- 0 273 565** **EP-A- 0 629 648**
**US-A- 5 292 849** **US-A1- 2004 073 031**

- **PATENT ABSTRACTS OF JAPAN vol. 1999, no. 11, 30 September 1999 (1999-09-30) & JP 11 158282 A (TOAGOSEI CO LTD), 15 June 1999 (1999-06-15)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 838 758 B1

## Description

### Related Application

This application claims priority under 35 U.S.C. §119 of U.S. Application Serial No. 60/637,861 filed December 20, 2004, and of Swedish Patent Application No. 0403093-8, filed on December 20, 2004, both of which are incorporated by reference in their entirety herein.

### Field of the Invention

The present invention relates to polysiloxanes having at least one terminal hydroxyalkyl group. In one embodiment, the polysiloxanes have refractive indices of 1.40-1.45 and densities above 1 $g/cm^3$. Further, the present invention also relates to the syntheses of such polysiloxanes, and to injectable ophthalmic compositions containing at least one polysiloxane and suitable for use in forming an intraocular lens.

### Background of the Invention

The human eye is a highly evolved and complex sensory organ. It is composed of a cornea, or clear outer tissue, which refracts light rays en route to the iris, the iris, which controls the size of the pupil and thus regulates the amount of light entering the eye, and a lens, which focuses the incoming light through the vitreous fluid onto the retina. The retina converts the incoming light into electrical energy, which is transmitted through the brain stem to the occipital cortex resulting in a visual image. In a perfect eye, the light path from the cornea through the lens and vitreous fluid to the retina is unobstructed. Any obstruction or loss in clarity within these structures causes scattering or absorption of light rays, which results in a diminished visual acuity. For example, the cornea can become damaged, resulting in edema, scarring or abrasions; the lens is susceptible to oxidative damage, trauma and infection; and the vitreous fluid can become cloudy due to hemorrhage or inflammation.

As an individual ages, the effects of oxidative damage caused by environmental exposure and endogenous free radical production accumulate, resulting in a loss of lens flexibility and denatured proteins that slowly coagulate, thereby reducing lens transparency. The natural flexibility of the lens is essential for focusing light onto the retina by a process referred to as accommodation. Accommodation allows the eye to automatically adjust refractive power for viewing objects at different distances. When the cumulative effects of oxidative damage diminish this flexibility, thus reducing near vision ability, it is known as presbyopia. Presbyopia usually begins to occur in adults during their mid-forties. An individual with presbyopia need spectacles of different powers for different object distances, or alternatively spectacles or contact lenses that have multifocal or progressive optics. All these alternatives have limitations in many practical situations.

Lenticular cataract is a lens disorder resulting from further development of coagulated protein. There are four common types of cataracts: senile cataracts associated with aging and oxidative stress; traumatic cataracts that develop after penetrating or non-penetrating impacts of objects on the eye or following exposure to intense radiation; complicated cataracts that are secondary to diseases such as diabetes mellitus or eye disorders such as detached retinas, glaucoma and retinitis pigmentosa; and toxic cataracts resulting from medicinal or chemical toxicity. Regardless of the cause, the disease results in impaired vision and may lead to blindness.

Treatment of cataract and/or presbyopia requires surgical removal of the lens, involving phacoemulsification followed by irrigation and aspiration. Without a lens, the eye is unable to focus the incoming light onto the retina. Consequently, an artificial lens is used to restore vision. Three types of prosthetic lenses are available: cataract spectacle lenses, external contact lenses and intraocular lenses (IOLs). Intraocular lenses, which can be either monofocal or multifocal, are currently used in the majority of cases to overcome the difficulties associated with cataract spectacle lenses and contact lenses. Multifocal intraocular lenses provide pseudo-accommodation, i.e. both distant and near objects can be seen sharply, however, there is always a contrast reduction in comparison with monofocal lenses. Multifocal lenses are sometimes used in cases of presbyopia without cataract, so-called clear lens exchange, despite the reduction in contrast.

IOLs mentioned in the prior art literature usually belong to one of the following categories: rigid, foldable, expansive hydrogels and injectable. The earliest IOLs coming into surgical practice were rigid implants, composed of poly(methyl methacrylate). These types of lenses require a large corneal incision, which resulted in protracted recovery times and the likelihood of introducing astigmatism. In an effort to reduce recovery time and patient discomfort, several small incision techniques in conjunction with intraocular lenses implantable through these small incisions have been developed.

Present IOLs, which are designed for small incision implantation, have elastomeric characteristics and are made of soft silicone or acrylic rubbers, or soft hydrogel materials. These types of lenses can be rolled or folded, inserted

into the capsular bag, and then unfolded once inside, but they typically provide no accommodative ability. However, there exist so-called accommodative lenses, which are claimed to provide accommodative ability by moving anteriorly in response to ciliary muscle contraction but the optical effect is minimal, about 1 diopter, corresponding to an object distance of 1 meter.

To further develop IOLs and reduce the size of the surgical incisions, techniques using injectable IOLs have been suggested. In these techniques, a low viscosity lens material is directly injected into the emptied capsular bag and cured *in situ* as a part of the surgical procedure. In such a process, the capsular bag is used as a mold to form the shape of the lens and thereby contribute to the control of its refraction. There have been several attempts to develop materials suitable for use as injectable IOLs as disclosed in US patents Nos. 5,278,258, 5,391,590, 5,411,553 and 5,476,515, and WO 01/76651.

The technique of cataract extraction and replacement of the natural lens for an accommodating IOL, i.e. an artificial crystalline lens (ACL), involves injection of a liquid having sufficiently low viscosity through a small incision into the capsular bag, followed by crosslinking of the liquid to create a lens of the required shape, using the form of the capsular bag as the mold. To reproduce the optical performance of the natural lens, the replacement lens will require a refractive index of about 1.42, and to respond to the accommodating forces, the compression modulus (Young's modulus) of the lens should be in the range of 1-5 kPa or less. Most researchers, e.g. Haefliger et al. (1994), J. Refractive and Corneal Surgery 10, 550-555, in the field of ACLs have used silicone-derived systems for filling the capsular bag, either in the form of silicone oils or LTV (low temperature vulcanising) silicone elastomers. Such systems suffer from certain disadvantages in the context of lens refilling in that lenses resulting from dimethyl silicones exhibit a restricted refractive index (1.40). Moreover, the LTV silicone elastomers cure slowly; up to 12 hours may be needed to complete their setting. This slow setting results in material loss from the capsular bag through the corneal incision.

Photo-polymerization provides an attractive alternative for increasing the curing rate and thereby reducing the treatment time. Hettlich et al., J. Cataract Refract. Surg. 1994, 20, 115-123, and de Groot et al., J. Biomacromolecules 2003, 4, 608-616, describe photocurable systems for making artificial lenses. However, the materials disclosed had too low of a density and/or too low and/or too high of a refractive index (which could cause myopia or hyperopia) for being suitable since the artificial lens used for replacing the natural lens should have a refractive index (RI) close to the RI of the natural lens, i.e. said lens should have a RI of 1.40-1.45.

Silicone materials having the above-mentioned characteristics suitable for injectable IOL, i.e. fast curing, a density higher than water, a refractive index close to the human lens and a sufficient elasticity able to accommodate, can be obtained by copolymerising different siloxane monomers. $\alpha,\omega$-Dihydroxyhexyl-silicones have been proven to be suitable intermediates to facilitate the transformation of the polysiloxanes to photocurable acryloyl esters since the hydroxyhexyldimethylsiloxy group has the adequate stability, Yilgor et al, Polym. Bull. 1998, 40, 525-532. However, the relatively high reactivity of the hydroxyl groups can cause several problems and therefore Kojima et al, J. Polym. Sci., A-1 1966, 4, 2325-2327, used sulphuric acid-catalysed polymerisation of octamethylcyclo-tetrasiloxane in the presence of 1,3-bis(4-hydroxybutyl)tetramethyldisiloxane in earlier studies on the synthesis of $\alpha,\omega$-dihydroxyl terminated siloxanes. The molecular weights of the obtained oligomers were several times higher than the expected values due to the dehydration caused by the sulphuric acid, which resulted in a loss of end-group functionality. Yilgör et al. have performed a controlled synthesis of $\alpha,\omega$-dihydroxyl terminated siloxanes via trifluoroacetic acid catalysed equilibration reaction of octamethylcyclotetrasiloxane and 1,3-bis(4-hydroxybutyl)-tetramethyldisiloxane (as an end capper), which was followed by mild hydrolysis of the terminal trifluoroacetyl groups, Reactive Oligomers, ACS Symposium Series 282; Harris et al; ACS: Washington D.C., USA, 1985; pp 161-174.

However, there exits still a need for a process for preparing well-defined, end-functional siloxanes which have a combination of controlled molecular weights and stable and easily and rapidly photo-crosslinked end-groups.

EP 629 648, US 5,292,849 and US 2007/073031 disclose hydroxyalkyl-terminated polysiloxanes or acryl-terminated polysiloxanes, but without any guidance to obtain both a specified high density and a high refractive index.

## Summary of the Invention

The present invention relates to hydroxyalkyl-terminated polysiloxanes, more specifically to, hydroxyalkyl terminated poly(dimethyl-co-diphenyl-co-methyltrifluoropropyl siloxanes). The present invention also relates to processes for preparing such copolymers and to compositions comprising them. Furthermore, the present invention also relates to methods of producing intraocular lenses by using said copolymers.

The polysiloxanes according to the present invention are advantageous in exhibiting a desirable combination of properties. For example, in one embodiment, the polysiloxanes exhibit controlled molecular weights and are stable and easily and rapidly photo-crosslinked. Further, the polysiloxanes can provide a polymerized composition in the form of a lens having a refractive index in the range of the human eye and a density greater than one.

These and additional advantages will be more further apparent in view of the following detailed description.

**Brief Description of the Drawings**

The following detailed description will be more fully understood in view of the drawings in which

Fig. 1 sets forth a diagrammatic relation of the predicted density of a terpolymer by volume fraction of the respective monomers;

Fig. 2 sets forth a diagrammatic relation of the predicted refractive index of a terpolymer by volume fraction of the respective monomers;

Fig. 3 sets forth a schematic reaction of base-catalyzed polymerization followed by trifluoroacetic acid redistribution and hydrolysis; and

Fig. 4 sets forth $^1$H-MNR spectra of a crude polymerization product.

**Detailed Description of the Invention**

The present invention is directed to polysiloxane copolymers according to claim 1 suitable for use, for example, in the field of injectable intraocular lenses. Advantageously, the invention provides a controlled synthesis method to prepare hydroxyalkyl-terminated polysiloxanes having refractive indices in the range of the human eye, densities higher than 1, a controlled molecular weight and stable end-groups, which end-groups are easily and rapidly crosslinked.

The present invention provides linear polysiloxane copolymers having at least one terminal hydroxyalkyl having densities higher than 1 g/cm$^3$ and refractive indices close to the RI of the human lens.

In one embodiment, the present invention provides linear polysiloxane copolymers having at least one terminal hydroxyalkyl group, which are prepared by a process comprising a combination of a base-catalysed polymerisation and an acid-catalysed redistribution.

According to one embodiment of the present invention, the at least one terminal hydroxyalkyl group is selected from the group consisting of hydroxyethyl, hydroxypropyl, hydroxybutyl, hydroxypentyl, hydroxyhexyl, hydroxyheptyl and hydroxyoctyl. According to a specific embodiment, the terminal group is hydroxyhexyl.

**[0023]** , The linear polysiloxane copolymers have the general formula of:

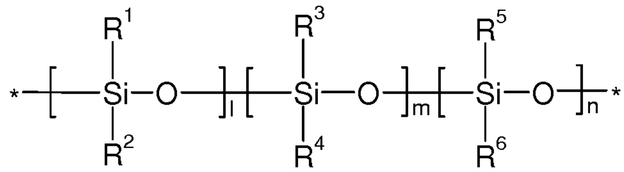

wherein $R^1$, $R^2$ and $R^6$ are independently $C_1$-$C_6$ alkyl; $R^3$ and $R^4$ are independently phenyl or $C_1$-$C_6$ alkyl; and $R^5$ is $CF_3(CH_2)_x$ wherein x is 1-5. Further, 1 is in the molar fraction range of 0 to 0.9; m is in the molar fraction range of 0 to 0.6; and n is in the molar fraction range of 0 to 0.6. In a specific embodiment, the linear polysiloxane copolymer is a terpolymer of the above formula in which $R^1$, $R^2$ and $R^6$ are methyl; $R^3$ and $R^4$ are phenyl; and $R^5$ is 3,3,3-trifluoropropyl. The general formula shown above shall be interpreted as a general formula, as the obtained copolymers could be randomly distributed copolymers, block copolymers, etc. In a specific embodiment, the obtained copolymers have a random distribution of monomers.

In another embodiment, the present invention provides an injectable ophthalmic composition suitable for forming an intraocular lens in the capsular bag of an eye. The composition comprises at least one of the above disclosed copolymers in which the terminal hydroxyalkyl groups have been converted to functional groups, for example acrylalkyl groups. The injectable ophthalmic composition can also comprise a medically acceptable photoinitiator, for example a UV-photoinitiator or a blue light photoinitiator. Further, the injectable composition can also comprise a UV absorber or other additive commonly used in ophthalmic injectable compositions. According to another embodiment of the present invention, the injectable ophthalmic composition comprises a mixture of di-functional and mono-functional copolymers of the copolymers disclosed above and non-functional copolymers having essentially the same structure as the functional copolymers, without the terminal hydroxyalkyl groups having been converted to functional groups. The compositions desirably should be able to pass through a 21 Gauge needle and must therefore in a specific embodiment have a viscosity of less than about 60,000 cSt, and in a more specific embodiment, the viscosity is less than 8,000 cSt. In order to reduce physiological hazards, only acryl-substituted siloxane polymers are introduced into the capsular bag together with the medically acceptable photoinitiators and optionally other components. Any suitable photoinitiator for in situ use may be employed. In one embodiment, the blue light photoinitiator is selected from a group comprising compounds derived from acyl phosphine oxides, bisacylphosphine oxides and titanocene photoinitiators. One of ordinary skill in the art can find other suitable compounds usable as photoinitiators, which fall within the scope of the present invention. However, the

photoinitiators used must be able, when exposed to blue light or UV, to initiate the photopolymerization of the acryl groups and in a specific embodiment they must be "photobleaching".

Further, another embodiment of the present invention provides a process for preparing the copolymers, using a combination of a base-catalysed polymerisation and an acid-catalysed redistribution. According to one specific embodiment, the copolymers are synthesised by using ring-opening polymerisation followed by a base-catalyzed polymerization using 1,3-bis(6-hydroxyhexyl)-1,1,3,3-tetramethyldisiloxane as an end-blocker. The hydroxyalkyl group, especially the hydroxyhexyl group, exhibits adequate stability in acidic medium. Thus, a base-catalyzed polymerization is combined with an acid-catalyzed redistribution, i.e. an esterification is combined with an acidic hydrolysis in which the terminal hydroxyalkyl groups are then freed. The obtained $\alpha,\omega$-dihydroxyl polymers are then acrylated to be suitable for an ophthalmic composition, i.e. in compositions that are, by photo-curing, used for producing intraocular lenses.

In another embodiment according to the present invention, an alternative method to synthesize a hydroxyhexyl-terminated siloxane terpolymer is provided. The method uses 1,3-bis(6-trimethylsiloxyhexyl)-1,1,3,3-tetramethyldisiloxane as an end-capper instead of 1,3-bis(6-hydroxyhexyl)-1,1,3,3-tetramethyldisiloxane in base-catalyzed ring-opening polymerization. Thus, the ring opening polymerization and the functionalization of the copolymer occurs in the same step, and is then followed by an acidic hydrolysis to free the terminal hydroxyl.

Yet another alternative embodiment for synthesizing hydroxyhexyl terminated terpolymers uses 1,3-bis (6-trimethylsiloxyhexyl)tetramethyldisiloxane (TMS) of the formula:

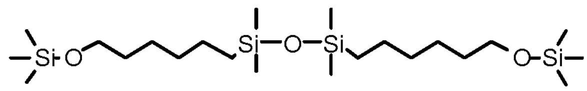

In this synthesis, the released trimethylsilanol will condense and form hexamethyldisiloxane and the hydroxyl-end will enter into the backbone. The terminal hydroxyl groups can be freed by using acidic hydrolysis. The terminal silanol group can be capped by both hydroxyalkyl and trimethylsilyl groups thus giving mixed end-capped copolymers, examples of which are of the formula:

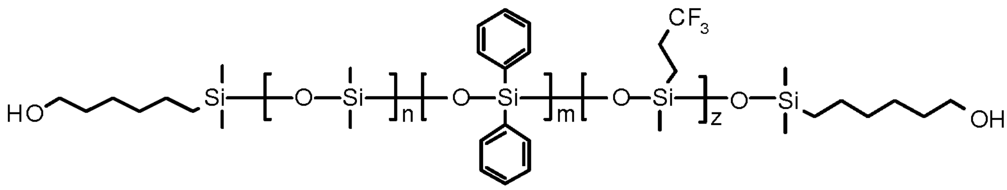

Bis(hydroxyhexyl)-terminated ter-polymer

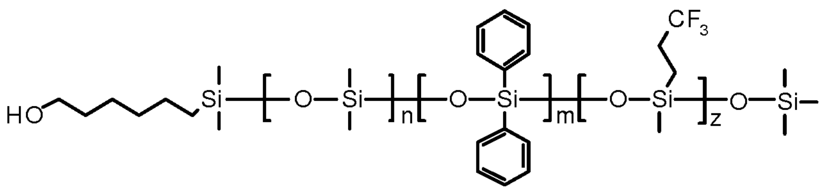

Hydroxyhexyl-trimethylsilyl-terminated ter-polymer

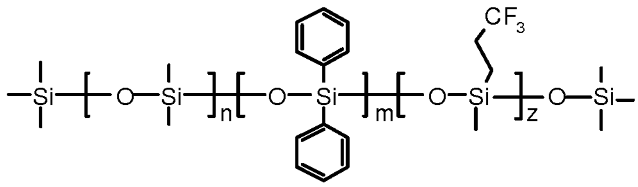

Bis(trimethylsilyl)-terminated ter-polymer

GPC viscosimetric results for mixture of bis(hydroxyhexyl)-, hydroxylhexyl/trimethylsilyl-, and bis(trimethylsilyl)-terminated poly(dimethyl-co-diphenyl-co-methyltrifluoropropylsiloxane) from such a process are set forth in Table 1:

**Table 1**

| Sample Code | 3 kD | 5 kD | 10 kD |
|---|---|---|---|
| Mn theory* [g · $mol^{-1}$] | 2740 | 5250 | 10240 |
| Mn [g · $mol^{-1}$] | 2810 | 4830 | 9385 |
| Mp [g · $mol^{-1}$] | 4470 | 11300 | 22150 |
| Mv [g · $mol^{-1}$] | 4740 | 11825 | 20600 |
| Mw [g · $mol^{-1}$] | 6110 | 13000 | 25225 |
| Mz [g · $mol^{-1}$] | 12110 | 28920 | 61780 |
| Mw/Mn | 2.18 | 2.69 | 2.69 |
| [η][ml · $g^{-1}$] | 4.03 | 11.08 | 15.40 |
| Log K | -1.309572 | -1.44487 | -1.35953 |
| A (MH) | 0.5295 | 0.6112 | 0.5904 |

***One mole of TMS is considered to correspond to two moles of terminal groups.**

Another embodiment of the present invention provides intraocular lenses comprising the inventive copolymers of the inventive compositions. The intraocular lenses have a refractive index close to the human lens, preferably 1.40-1.45, and are preferably accommodative, i.e. they will have such an elasticity modulus that they may accommodate in the capsular bag using the ciliary muscles.

Further, another embodiment of the present invention is a method for preparing an intraocular lens in the capsular bag of the eye. The method comprises the steps of preparing a terminal hydroxyalkyl group copolymer according to the present invention, converting the at least one terminal hydroxyalkyl group of the copolymer to at least one acrylalkyl group, mixing the obtained copolymer and a medically acceptable photo initiator to obtain an injectable ophthalmic composition, injecting the composition into an empty capsular bag of an eye, and then initiating photopolymerisation and thus producing an intraocular lens in the capsular bag of the eye. The capsular bag of the eye will thus function as a mold. According to one embodiment according to the present invention, the intraocular lens is produced using a composition comprising mixed end-capped polymers, i.e. bis(hydroxyalkyl)-, hydroxylalkyl-trimethylsilyl-, and bis(trimethylsilyl)-terminated terpolymers.

Another embodiment of the present invention is a surgical kit comprising the inventive compositions.

EXAMPLES

The following examples are included in order to illustrate the principles of the present invention and should not in any way be interpreted as limiting the scope of the invention. It is to be understood that the compositions used for producing lenses can further comprise conventional constituents such as, but not limited to, cross-linking agents and UV-absorbers.

A combination of octamethylcyclotetrasiloxane with the high-density monomer (3,3,3-trifluoropropyl)methyl cyclotrisiloxane and the high-refractive-index monomer octaphenylcyclotetrasiloxane will give an adjustment of refractive index and density, which is necessary due to the intended use (as an intraocular lens) and for obtaining a copolymer having a density higher than 1 (which will avoid flotation of the material during surgery). $\alpha,\omega$-Dihydroxylhexyl-terminated polysiloxane can be transformed to polysiloxane with different functionalities, for example, acryloyl-ended and photocurable precursors. A common polymerization procedure for introduction of hydroxyl to siloxane involves the use of trifluoroacetic acid that catalyses the ring opening equilibration while protecting the terminal hydroxyls by formation of an ester.

Acrylation, using acryloylchloride, of the hydroxyhexyl terminated polysiloxane gives acryloyl-terminated terpolymers. These terpolymers can be photocured at room temperature with the aid of a blue light photoinitiator and will form an elastic, transparent, colorless gel and thus can be used for injectable intraocular lenses, which are cured in the capsular bag of the eye.

The experiments, which will be presented herein, can be summarized as follows: the ratios of octamethylcyclotetrasiloxane ($D_4$), octaphenyl-cyclotetrasiloxane ($D''_4$) and (3,3,3-trifluoro-propyl) methylcyclotrisiloxane ($F_3$) are varied, whereby the refractive indices and densities of the siloxane terpolymers are modulated. Predictions of the refractive index and density with different compositions are made using equations 1 and 2:

$$n_{20}^{d} = 1.404 v_{D4} + 1.64 v_{D''4} + 1.383 v_{F3} \qquad (1)$$

$$d_4^{20} = 0.95v_{D4} + 1.185v_{D''4} + 1.24v_{F3} \qquad (2)$$

wherein $n_{20}^{d}$ and $d_4^{20}$ are the refractive index and density of the terpolymer, $v_{D4}$, $v_{D''4}$, $v_{F3}$ are the volume fractions of $D_4$, $D''_4$ and $F_3$ respectively. The volume fractions are calculated from the molar volumes of the monomers. The contribution of the end-blocker is neglected. The numerical coefficients are the refractive indices and densities of the monomers, with the exception of the refractive index for $D''_4$, which is a value derived from a commercial poly(dimethyl-co-diphenylsiloxane). The relation of the density with volume fraction of $D_4$, $D''_4$ and $F_3$ is presented in Fig. 1 while a plot of refractive index as a function of volume fraction is shown in Fig. 2. Modulation of the ratio of these three monomers gives terpolymer having the desired refractive index range (1.40-1.45) and density (>1.000). In some cases, substantially more than 2 mol% of $D''_4$ is included into the terpolymer by co-polymerisation in order to obtain the desired properties.

By combining the base-catalysed polymerisation with acid-catalysed redistribution, a $\alpha,\omega$-bis(trifluoroacetylhexyl) terminated terpolymer is obtained. In the first step, the three monomers $D_4$, $D''_4$ and $F_3$ are co-polymerised without end-capper using tetramethylammonium hydroxide as a catalyst. In the second step, the terpolymer is cleaved by trifluoroacetic acid catalysed redistribution in the presence of the end-capper, 1,3-bis(6-hydroxyhexyl)-tetramethyldisiloxane, as shown in Fig. 3. In the second step, trifluoroacetic acid has two functions, it will both catalyse the redistribution reaction and form trifluoro-acetate with the free hydroxyhexyl group, thus protecting it. The hydroxyhexyl incorporation into the polysiloxane main chain was proved using $^1$H-NMR (Fig. 4). Fig. 4 shows H-NMR (400 MHz) of the reaction mixture and the hydrolysed samples of hydroxyhexyl end-capped poly(dimethyl-co-diphenyl-co-methyltrifluoropropylsiloxane) in dichloromethane-$d_2$. The abscissa unit is parts per million, i.e. ppm. The $^1$H-NMR spectra of the crude polymerisation product shows three relatively strong triplets at $\delta$ 3.71, 3.66 and 3.61 ppm. The triplet at $\delta$ 3.71 ppm indicates that the hexanol end group enters into the main chain by attaching to the diphenyl siloxane unit. The peaks at 3.66 and 3.61 ppm represent the attachment of the hexanol to methyltrifluoropropylsiloxane and dimethylsiloxane units, respectively. These chemical shifts are verified in model reactions using butanol- $D_4$, butanol- $F_3$ and butanol- $D_4$ - $D''_4$ systems under identical conditions without gelation. The ratio of integral values, triplet, $J$ 6.3, HO-C$\underline{H_2}$- on the $^1$H-NMR spectra is 6 : 12 : 80, corresponding to the molar ratio of the three different building blocks of the polymer.

The silicon-oxygen-carbon bond in the end-capped chain exhibits higher reactivity in acidolysis than oxygen-silicon-oxygen bond in siloxane backbone. The hydroxyalkyl groups are therefore readily freed during acid-catalysed hydrolysis in THF or dichloromethane using only a trace amount of hydrochloric acid.

GPC is used to determine the molecular weight of the hydroxyhexyl-terminated terpolymer before and after the hydrolysis with results shown in Table 1.

*The Experiments*

Materials: All chemicals and solvents are obtained from Fluka and Aldrich and used without purification except for octamethylcyclotetrasiloxane ($D_4$), (3,3,3-trifluoro-propyl) methylcyclotrisiloxane ($F_3$), which is distilled at reduced pressure and octaphenyl-cyclotetrasiloxane ($D''_4$), which is purified by recrystallization from dichloromethane. 1,3-bis(6-hydroxyhexyl) 1,1,3,3-tetramethyldisiloxane and 1,3-bis(6-trimethylsiloxyhexyl) tetramethyldisiloxane (TMS) are prepared according to Merker et al, J. Polym. Sci. 1960, 43, 297-310. Polystyrene from Polymer Laboratories (Shropshire, UK) is used as narrow standards with a polydispersity index < 1.05.

Instruments: Proton NMR spectra are recorded in dichloromethane-$d_2$ on a Bruker DPX-400 instrument using residual $CH_2Cl_2$ ($\delta$ = 5.25 ppm) as an internal reference. Abbreviation of the NMR data is, *t* for triplet, *bs* for broad singlet, *s* for singlet. FTIR spectra are obtained in attenuated total reflection (ATR) mode on a Nicolet Magna DSP 650 equipped with the Golden Gate® accessory and corrected for the wavelength-dependence of the penetration depth. GPC is performed on a Waters 150CV modified with on-line differential viscometry. THF is used as eluent on three Waters high-resolution columns: HR/4/3/2, at 40°C. The universal values are calculated from the viscosity detector curves using polystyrene standards. The amount of injected polymer is 0.1 ml at a concentration of 5 mg/ml throughout. The refractive indices are determined on a B+S RFM 340 refractrometer.

*Example 1: Synthesis of trifluoroacetyl-hexyl terminated poly(dimethyl-co-diphenyl-co-methyltrifluoropropyl-siloxane) (I).*

A Schlenk balloon (100 ml) equipped with a mechanical stirrer is charged with $D_4$ (8.45 g, 28.5 mmol), $F_3$ (2.12 g, 4.52 mmol), and $D''_4$ (1.62 g, 2.04 mmol) in THF (2 ml) and tetramethylammonium hydroxide pentahydrate (90 mg) is used as catalyst. The reactor is flushed with nitrogen and heated to 110 °C to initiate polymerization. The molecular weight and consequently the viscosity becomes high after two days of reaction, which causes stirring problems. At this point, the temperature is raised to 160 °C in 20 min in order to decompose the catalyst. The reaction mixture is cooled

to 60 °C, and 1,3-bis(6-hydroxy-hexyl)-1,1,3,3-tetramethyldisiloxane (0.81 g, 2.43 mmol) as end-blocker and trifluoroacetic acid (1.14 g, 10 mmol, 0.8 ml) are added in 2 ml THF. After 24 h of stirring at 60 °C, the mixture is dissolved in 50 ml diethyl ether, washed with water (2 × 100 ml) and dried with sodium sulphate. The solvent is evaporated and the $\alpha,\omega$-bis(trifluoroacetylhexyl) terminated terpolymer is recovered as a colorless oil (12.0 g, 91%). $^1$H-NMR (400 MHz, $CD_2Cl_2$, ppm): $\delta$ 7.65 (3.8 H, bs, *o*-phenyl), 7.38 (5.6 H, m, *m*-, *p*-phenyl), 4.38 (0.7 H, t, *J* 6.7, $F_3CCO$-O-$CH_2$-), 2.2-1.9 (4.3 H, m, -Si-$CH_2$-$\underline{CH_2}$-$CF_3$), 1.56 (2.5 H, s, -$CH_2$-), 1.4-1.3 (3.0 H, m, -$CH_2$-), 0.84-0.50 (4.3 H, m, -Si-$\underline{CH_2}$-$CH_2$-$CF_3$ and -$CH_2$-Si-), 0.21-0.06 (100 H, m, -Si-$CH_3$), $n_d^{20}$ 1.4237.

*Example 2: Synthesis of hydroxyhexyl-terminated poly(dimethyl-co-diphenyl-co-methytrifluoropropyl-siloxane) (II) via hydrolysis of I.*

Trifluoroacetyl-hexyl terminated terpolymer (5 g) is dissolved in 30 ml THF and an aqueous solution of sodium carbonate (2.5%, 30 ml) is added. The biphasic mixture is heated to 60°C and vigorously stirred for 48 h followed by separation of the two phases. The organic phase is dried with sodium sulphate and magnesium sulphate in turn. The solvent is removed under vacuum, resulting in viscous oil (9.6 g, 96%). $^1$H-NMR (400 MHz, $CD_2Cl_2$, ppm): $\delta$ 7.65 (3.9 H, bs, *o*-phenyl), 7.38 (5.7 H, m, *m*-, *p*-phenyl), 2.2-1.9 (4.4 H, m, -Si-$CH_2$-$C\underline{H}_2$-$CF_3$), 0.84-0.63 (3.6 H, m, -Si-$C\underline{H}_2$-$CH_2$-$CF_3$), 0.21-0.06 (100 H, m, -Si-$C\underline{H}_3$), $n_d^{20}$ 1.4263. The aqueous phase is extracted with diethyl ether (3 × 20 ml) and the combined ethereal layers are dried with sodium sulphate and magnesium sulphate. After the solvent is stripped off under vacuum, a trace of colorless oil is recovered (0.3 g, 3%).

Table 2 sets forth GPC viscosimetric results for hydrolysed and non-hydrolysed end-capped poly(dimethyl-co-diphenyl-co-methyltrifluoropropylsiloxane):

*Table 2*

| Sample Code | 5 kD | | 10 kD | |
|---|---|---|---|---|
| | Before hydrolysis | After hydrolysis | Before hydrolysis | After hydrolysis |
| Mn theory* [g · mol$^{-1}$] | 5180 | 5180 | 9700 | 9700 |
| Mn [g · mol$^{-1}$] | 5730 | 2845 | 17840 | 4110 |
| Mp [g · mol$^{-1}$] | 30700 | 6450 | 153260 | 9835 |
| Mv [g · mol$^{-1}$] | 26420 | 7190 | 100440 | 9890 |
| Mw [g · mol$^{-1}$] | 41300 | 9680 | 159930 | 12770 |
| Mz [g · mol$^{-1}$] | 140890 | 22130 | 556110 | 27900 |
| Mw/Mn | 7.21 | 3.40 | 8.96 | 3.11 |
| [$\eta$][ml · g$^{-1}$] | 12.19 | 5.23 | 34.59 | 6.35 |
| Log K | -0.99069 | -0.9202 | -0.84513 | -1.03548 |
| A (MH) | 0.4697 | 0.4248 | 0.4766 | 0.4602 |

*One mole of TMS is considered as two moles of terminal groups

*Example 3: Synthesis of II via 1,3-bis(6-hydroxyhexyl)-1,1,3,3-tetramethyldisiloxane as end-blocker.*

A Schlenk balloon (100 ml) equipped with an overhead stirrer is charged with $D_4$ (8.45 g, 28.5 mmol), $F_3$ (2.12 g, 4.53 mmol), $D''_4$ (1.62 g, 2.04 mmol) and 1,3-bis(6-hydroxyhexyl)-1,1,3,3-tetramethyldisiloxane (0.41 g, 1.23 mmol) (which is used as end-blocker) in 2 ml THF and tetramethylammonium hydroxide pentahydrate (90 mg) (which is used as catalyst). The reactor is heated to 110 °C and flushed with nitrogen. The mixture is cooled after 12 h of reaction to room temperature since longer reaction time causes gelation. Hydrochloric acid (0.2 ml 35%) in 10 ml THF is added to liberate the hydroxyl end-group via hydrolysis or protonation. The hydrolysis is monitored by means of an ATR-IR spectrometer. At the end of the hydrolysis (60 min) the silicone oil is taken up with 50 ml diethyl ether and extracted with water (2 × 100 ml) and is then washed with brine (30 ml). The ethereal layer is dried with magnesium sulphate, filtrated and stripped off under vacuum to give 11.71g (92.6%) of a colorless oil. $^1$H-NMR (400MHz, $CD_2Cl_2$, ppm): $\delta$ 7.71-7.53 (4.4 H, bs, o-phenyl), 7.48-7.25 (6.8 H, m, m-, p-phenyl), 3.61 (0.7 H, t, J 6.3, HO-$C\underline{H}_2$-), 2.2-1.9 (3.8H, m, -Si-$CH_2$-$C\underline{H}_2$-$CF_3$), 1.56 (2.5 H, s, -$C\underline{H}_2$-), 1.4-1.3 (3.4 H, m, -$C\underline{H}_2$-), 0.84-0.63 (4.0 H, m, -Si-$C\underline{H}_2$-$CH_2$-$CF_3$), 0.59 (0.9 H, t, J 7.7, -$C\underline{H}_2$-Si-), 0.21-0.06 (100H, m, -Si-$C\underline{H}_3$), $n_d^{20}$ 1.4279.

*Example 4: Typical procedure of synthesis of II via 1,3-bis(6-trimethylsiloxylhexyl)-1,1,3,3-tetramethyldisiloxane as end-blocker.*

As described above, the three monomers: $D_4$ (18.4 g, 62.0 mmol), $F_3$ (5.1 g, 10.9 mmol), and $D''_4$ (4.5 g, 5.7 mmol) are charged into the reactor. 1,3-Bis(6-trimethylsiloxyl-hexyl)-1,1,3,3-tetramethyldisiloxane (2.7 g, 5.6 mmol) and tetramethylammonium hydroxide pentahydrate (120 mg) are added. The reactor is flushed with nitrogen and heated to 110°C and after 12 h of reaction, the mixture is cooled to room temperature and the polymer is taken up in a mixture of diethyl ether (50 ml) and methanol (10 ml). Aqueous hydrochloric acid (0.8 ml, 35%) is added in order to liberate the mixed end groups. After 1 hour of vigorous stirring, the mixture is extracted with 100 ml of methanol: water (1:1) and then with water until pH is equal to 7. The ethereal phase is dried with sodium sulphate and magnesium sulphate. The solvent is evaporated to afford the hydroxyhexyl terminated terpolymer (26.6 g, 86%). $^1$H-NMR is identical to the above synthesis, $n_d^{20}$ 1.4231.

*Example 5 Typical procedure of synthesis of acrylated poly(dimethyl-co-diphenyl-co-methytrifluoropropyl-siloxane) (III).*

A 50 ml 3-neck round-bottom flask equipped with a rubber stopper and a magnetic stir bar is charged with *II* (5.05 g, 1.01 mmol) in 13 ml $CH_2Cl_2$ and $CaH_2$ (254.4 mg, 6.04 mmol). The flask is closed with two glass stoppers and the solution is cooled to 0 °C with ice/water. Acryloyl chloride (0.3 ml, 3.39 mmol), in excess of the siloxane, is injected into the flask through the rubber stopper. The solution is stirred for 24 hr at room temperature and filtered through a P5 sintered glass filter. The colorless solution is transferred into a 100 ml separation funnel and washed with 2 $\times$ 10 ml of water. The solvent is removed a using rotary evaporator and vacuum oven, and an oil that is clear and colorless is obtained in a yield of 96 %. $^1$H-NMR (400MHz, $CDCl_3$, ppm): $\delta$ 7.63-7.51 (4.2 H, bis ortho-phenyl), 7.41-7.26 (6.2 H, m, meta-, para-phenyl), 6.41-5.79 (0.2 H, m, $C\underline{H}_2$=$C\underline{H}$-COO-), 4.14 (0.7 H, bs, -COO-$C\underline{H}_2$-), 2.12-1.95 (3.0 H, m, -Si-$CH_2$-$C\underline{H}_2$-$CF_3$), 1.34-1.31 (1.4 H, m, -$C\underline{H}_2$-), 1.23-1.22 (1.5 H, m, -$C\underline{H}_2$-), 1.06 (1.6 H, bs, -$C\underline{H}_2$-), 0.63-0.60 (1.2 H, m, -$C\underline{H}_2$-), 0.79-0.66 (2.91 H, m, -Si-$C\underline{H}_2$-$CH_2$-$CF_3$), 0.52 (0.9 H, m, -$C\underline{H}_2$-Si-), 0.22-0.1 (82.1 H, bm, -Si-$C\underline{H}_3$), $n_d^{20}$ 1.4339.

*Example 6: Photo-curing of III.*

0.02 g of bis(2,4,6-trimethylbenzoyl)-phenylphosphineoxide (Irgacure 819) and 1.0 g of *III* are dissolved in 10 ml of methylene chloride. The solvent is stripped off and the viscous liquid is transferred into a polystyrene cuvette. The curing is carried out with UV light (100 W high pressure Xe lamp, distance 20 cm, 1 minute irradiation).

*Example 7 Implantation of a composition comprising III*

A human cadaver eye is prepared with a small aperture incision into the capsular bag and the crystalline lens is removed. A silicon plug is used for preventing the composition from leaching.

1.0 g of III is dissolved together with a UV photoinitiator in 10 ml of methylene chloride. The solvent is stripped off and the obtained viscous liquid is suctioned by a conventional cannula and injected into the empty capsular bag. The composition is cured with UV-light and an intraocular lens is thus produced.

The specific embodiments and examples described herein are illustrious in nature only and are not intended to be limiting of the invention defined by the claims. Additional embodiments and examples of the various aspects of the invention defined by the claims and/or which are equivalent to the specific embodiments and examples set forth herein may be apparent to one of ordinary skill in the art and are included within the scope of the claimed invention.

## Claims

**1.** A linear polysiloxane copolymer having the general formula:

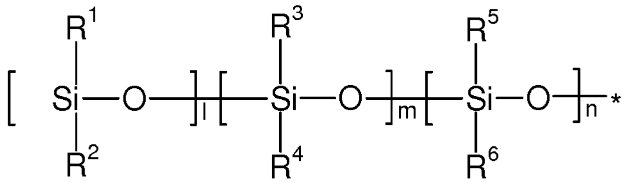

wherein $R^1$, $R^2$ and $R^6$ are independently $C_1$-$C_6$ alkyl; $R^3$ is phenyl; $R^4$ is phenyl or $C_1$-$C_6$ alkyl; $R^5$ is $CF_3(CH_2)_x$ wherein x is 1-5;1 is in the molar fraction range of 0 to 0.95; m is in the molar fraction range of 0 to 0.7; and n is in the molar fraction range of 0 to 0.65,
**characterized in that** the copolymer has at least one terminal hydroxyalkyl group, and **in that** the copolymer has a density higher than 1 g/cm$^3$ and a refractive index of 1.40-1.45.

**2.** A linear polysiloxane copolymer according to claim 1, wherein the copolymer is obtained by a process comprising a combination of a base-catalysed polymerisation with an acid-catalysed redistribution and wherein the copolymer has a density higher than 1 g/cm$^3$ and a refractive index of 1.40-1.45.

**3.** A linear polysiloxane copolymer according to claim 1, wherein the at least one terminal group is selected from the group consisting of hydroxyethyl, hydroxypropyl, hydroxybutyl, hydroxypentyl, hydroxyhexyl, hydroxyheptyl and hydroxyoctyl.

**4.** A linear polysiloxane copolymer according to claim 3, wherein the terminal group is hydroxyhexyl.

**5.** A linear polysiloxane copolymer according to claim 1, wherein the copolymer is a randomly distributed terpolymer in which $R^1$, $R^2$ and $R^6$ are methyl; $R^3$ and $R^4$ are phenyl; and $R^5$ is 3,3,3-trifluoropropyl.

**6.** A process for preparing a linear siloxane polymer according to claim 1, comprising

(a) polymerizing siloxane monomers in a base catalysed polymerization reaction;
(b) introducing terminal alkyl groups to the polymer of step (a) by an acid catalyst redistribution process; and
(c) hydrolyzing the polymer obtained in step (b) to yield hydroxyalkyl- terminated polysiloxane.

**7.** A process according to claim 6, wherein the polymerization of the siloxane monomers is a ring-opening polymerisation.

**8.** A process according to claim 7, wherein the ring-opening polymerization of the siloxane monomers and functionalization are performed in the same step of the process.

**9.** A process according to claim 6, wherein the acid catalyst redistribution process is performed in the presence of an 1,3-bis(6-hydroxyalkyl)-tetradimethyldisiloxane end-capper.

**10.** A method of producing an intraocular lens, comprising the steps of:

(a) preparing linear polysiloxane copolymer having at least one terminal hydroxyalkyl group by a process according to claim 6;
(b) converting the at least one terminal hydroxyalkyl group of said copolymer to at least one acrylalkyl group;
(c) mixing the copolymer obtained in step (b). with a medically acceptable photoinitiator;
(d) injecting the mixture obtained in step (c) into an empty capsular bag of an eye; and
(e) initiating a polymerisation and thus producing an intraocular lens in the capsular bag of the eye.

## Patentansprüche

**1.** Lineares Polysiloxancopolymer der allgemeinen Formel:

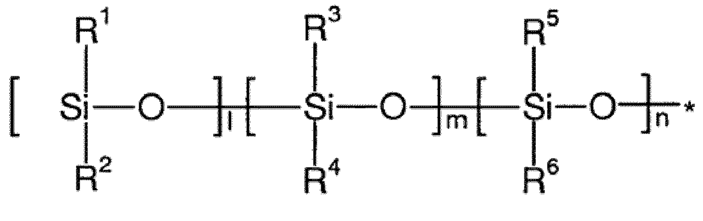

wobei $R^1$, $R^2$ und $R^6$ unabhängig $C_1$-$C_6$-Alkyl sind, $R^3$ Phenyl ist; $R^4$ Phenyl oder $C_1$-$C_6$-Alkyl ist; $R^5$ $CF_3(CH_2)$x ist, wobei x 1 - 5 ist; l im Molfraktionsbereich von 0 bis 0,95 liegt; m im Molfraktionsbereich von 0 bis 0,7 liegt; und n im

Molfraktionsbereich von 0 bis 0,65 liegt, **dadurch gekennzeichnet, dass** das Copolymer wenigstens eine terminale Hydroxyalkylgruppe aufweist, und dass das Copolymer eine Dichte von höher als 1 g/cm$^3$ aufweist und einen Brechungsindex von 1,40 - 1,45.

**2.** Lineares Polysiloxancopolymer nach Anspruch 1, wobei das Copolymer durch ein Verfahren umfassend eine Kombination aus einer basenkatalysierten Polymerisation mit einer säurekatalysierten Redistribution erhalten wird und wobei das Copolymer eine Dichte von höher als 1 g/cm$^3$ und einen Brechungsindex von 1,40 - 1,45 aufweist.

**3.** Lineares Polysiloxancopolymer nach Anspruch 1, wobei die wenigstens eine terminale Gruppe ausgewählt ist aus der Gruppe bestehend aus Hydroxyethyl, Hydroxypropyl, Hydroxybutyl, Hydroxypentyl, Hydroxyhexyl, Hydroxyheptyl und Hydroxyoctyl.

**4.** Lineares Polysiloxancopolymer nach Anspruch 3, wobei die terminale Gruppe Hydroxyhexyl ist.

**5.** Lineares Polysiloxancopolymer nach Anspruch 1, wobei das Copolymer ein unregelmäßig verteiltes Terpolymer ist, worin $R^1$, $R^2$ und $R^6$ Methyl sind; $R^3$ und $R^4$ Phenyl sind; und $R^5$ 3,3,3-Trifluorpropyl ist.

**6.** Verfahren zum Herstellen eines linearen Siloxanpolymers nach Anspruch 1, umfassend

(a) Polymerisieren von Siloxanmonomeren in einer basenkatalysierten Polymerisationsreaktion;
(b) Einführen von terminalen Alkylgruppen in das Polymer des Schritts (a) durch einen säurekatalysierten Redistributionsprozess; und
(c) Hydrolysieren des in Schritt (b) erhaltenen Polymers, um Hydroxylalkylterminiertes Polysiloxan zu ergeben.

**7.** Verfahren nach Anspruch 6, wobei die Polymerisation der Siloxanmonomere eine ringöffnende Polymerisation ist.

**8.** Verfahren nach Anspruch 7, wobei die ringöffnende Polymerisation der Siloxanmonomere und Funktionalisierung im gleichen Verfahrensschritt durchgeführt werden.

**9.** Verfahren nach Anspruch 6, wobei der säurekatalysierte Restributionsprozess in der Anwesenheit eines 1,3-Bis(6-hydroxyalkyl)-tetradimethyldisiloxan-Endcappers durchgeführt wird.

**10.** Verfahren zum Erzeugen einer Intraokularlinse, umfassend die Schritte:

(a) Herstellen eines linearen Polysiloxancopolymers mit wenigstens einer terminalen Hydroxyalkylgruppe durch ein Verfahren nach Anspruch 6;
(b) Umwandeln der wengistens einen terminalen Hydroxyalkylgruppe des Copolymers in wenigstens eine Acrylalkylgruppe;
(c) Mischen des in Schritt (b) erhaltenen Copolymers mit einem medizinisch verträglichen Photoinitiator;
(d) Injizieren der in Schritt (c) erhaltenen Mischung in einen leeren Kapselsack eines Auges; und
(e) Starten einer Polymerisation und somit Erzeugen einer Intraokularlinse im Kapselsack des Auges.

## Revendications

**1.** Copolymère de polysiloxane linéaire ayant la formule générale :

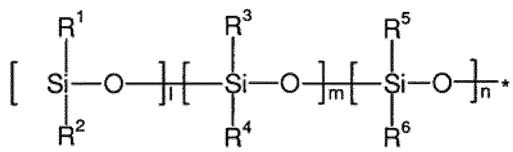

dans laquelle $R^1$, $R^2$ et $R^6$ représentent indépendamment un alkyle en $C_1$-$C_6$ ; $R^3$ représente un phényle ; $R^4$ représente un phényle ou un alkyle en $C_1$-$C_6$ ; $R^5$ est $CF_3(CH_2)_x$ dans laquelle x est 1-5 ; l se situe dans la plage de fraction molaire de 0 à 0,95 ; m se situe dans la plage de fraction molaire de 0 à 0,7 ; et n se situe dans la plage de fraction molaire de 0 à 0,65,
**caractérisé en ce que** le copolymère présente au moins un groupe hydroxyalkyle terminal, et **en ce que** le copolymère présente une densité supérieure à 1 $g/cm^3$ et un indice de réfraction de 1,40-1,45.

**2.** Copolymère de polysiloxane linéaire selon la revendication 1, **caractérisé en ce que** le copolymère est obtenu par un procédé comprenant une combinaison d'une polymérisation à catalyse basique avec une redistribution à catalyse acide et dans lequel le copolymère présente une densité supérieure à 1 $g/cm^3$ et un indice de réfraction de 1,40-1,45.

**3.** Copolymère de polysiloxane linéaire selon la revendication 1, **caractérisé en ce que** l'au moins un groupe terminal est choisi dans le groupe constitué d'un hydroxyéthyle, un hydroxypropyle, un hydroxybutyle, un hydroxypentyle, un hydroxyhéxyle, un hydroxyheptyle et un hydroxyoctyle.

**4.** Copolymère de polysiloxane linéaire selon la revendication 3, dans lequel le groupe terminal est un hydroxyhéxyle.

**5.** Copolymère de polysiloxane linéaire selon la revendication 1, dans lequel le copolymère est un terpolymère réparti au hasard dans lequel $R^1$, $R^2$ et $R^6$ représentent un méthyle; $R^3$ et $R^4$ représentent un phényle ; et $R^5$ représente le 3,3,3-trifluoropropyle.

**6.** Procédé de préparation d'un polymère de siloxane linéaire selon la revendication 1, comprenant

(a) la polymérisation de monomères de siloxane dans une réaction de polymérisation à catalyse basique ;
(b) l'introduction de groupes terminaux alkyles sur le polymère de l'étape (a) par un procédé de redistribution à catalyse acide ; et
(c) l'hydrolyse du polymère obtenu dans l'étape (b) pour donner un polysiloxane à terminaison hydroxyalkyle.

**7.** Procédé selon la revendication 6, dans lequel la polymérisation des monomères de siloxane est une polymérisation par ouverture de cycle.

**8.** Procédé selon la revendication 7, dans lequel la polymérisation par ouverture de cycle des monomères de siloxane et la fonctionnalisation sont réalisées dans la même étape du procédé.

**9.** Procédé selon la revendication 6, **caractérisé en ce que** le procédé de redistribution catalysée par un acide est réalisé en présence d'un agent de protection d'extrémités 1,3-bis (6-hydroxyalkyl)-tétradiméthyldisiloxane.

**10.** Procédé de fabrication d'une lentille intra-oculaire, comprenant les étapes :

(a) de préparation d'un copolymère de polysiloxane linéaire ayant au moins un groupe hydroxyalkyle terminal par un procédé selon la revendication 6 ;
(b) de conversion de l'au moins un groupe hydroxyalkyle terminal dudit copolymère en au moins un groupe acrylalkyle ;
(c) de mélange du copolymère obtenu à l'étape (b) avec un photoinitiateur médicalement acceptable ;
(d) d'injection du mélange obtenu à l'étape (c) dans un sac capsulaire vide d'un oeil ; et
(e) d'initiation d'une polymérisation et fabriquer ainsi une lentille intra-oculaire dans le sac capsulaire de l'oeil.

**Fig. 1**

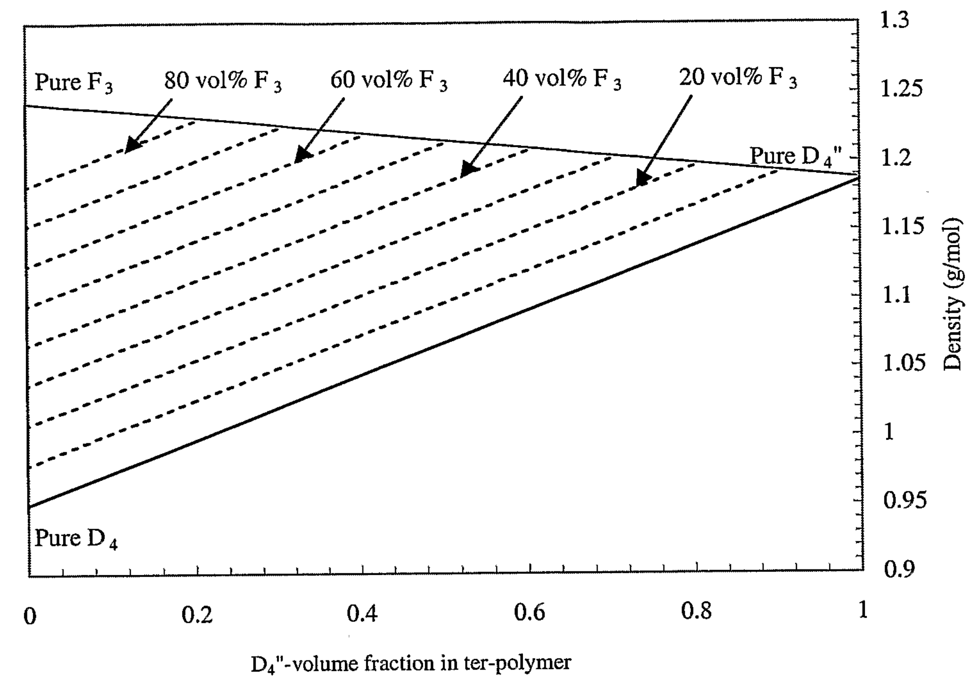
Pure F 3
80 vol% F 3
60 vol% F 3
40 vol% F 3
20 vol% F 3
Pure D 4"
Pure D 4
1.3
1.25
1.2
1.15
1.1
1.05
1
0.95
0.9
Density (g/mol)
0
0.2
0.4
0.6
0.8
1
D4"-volume fraction in ter-polymer

**Fig. 2**

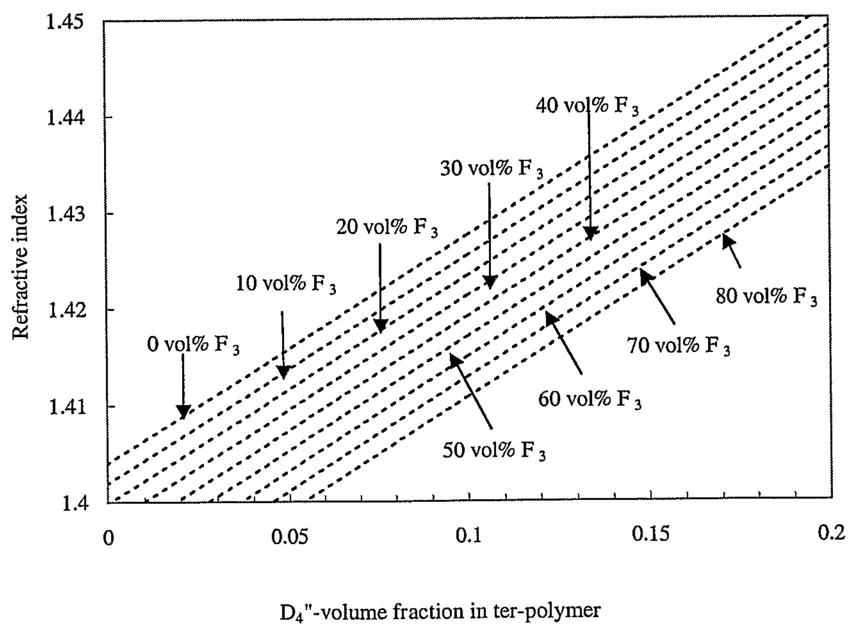
1.45
1.44
1.43
1.42
1.41
1.4
Refractive index
40 vol% F 3
30 vol% F 3
20 vol% F 3
10 vol% F 3
0 vol% F 3
80 vol% F 3
70 vol% F 3
60 vol% F 3
50 vol% F 3
0
0.05
0.1
0.15
0.2
D4"-volume fraction in ter-polymer

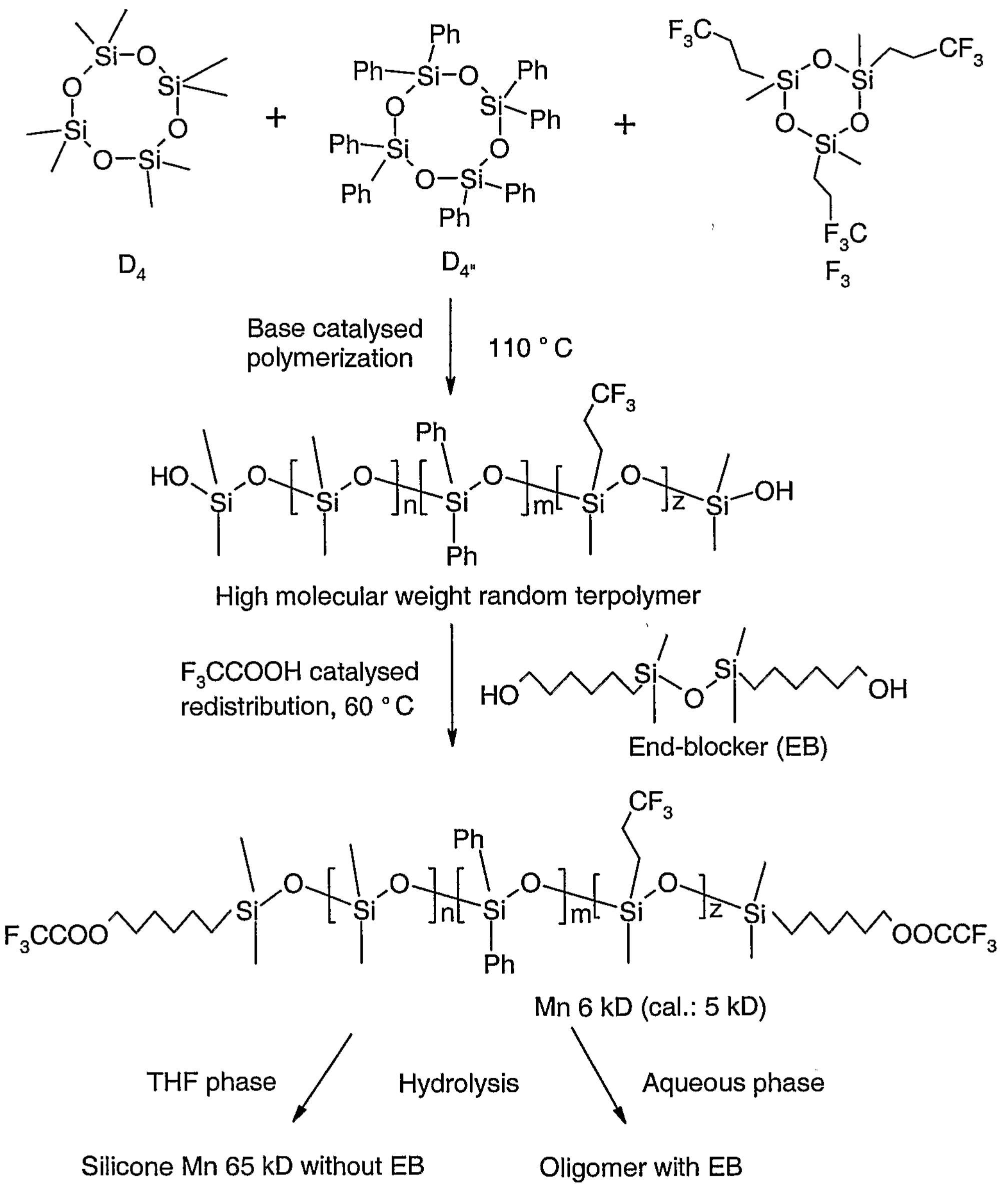
D$_4$
D$_{4''}$
Base catalysed polymerization
110 °C
High molecular weight random terpolymer
F$_3$CCOOH catalysed redistribution, 60 °C
End-blocker (EB)
Mn 6 kD (cal.: 5 kD)
THF phase
Hydrolysis
Aqueous phase
Silicone Mn 65 kD without EB
Oligomer with EB

**Fig. 3**

**Fig. 4**

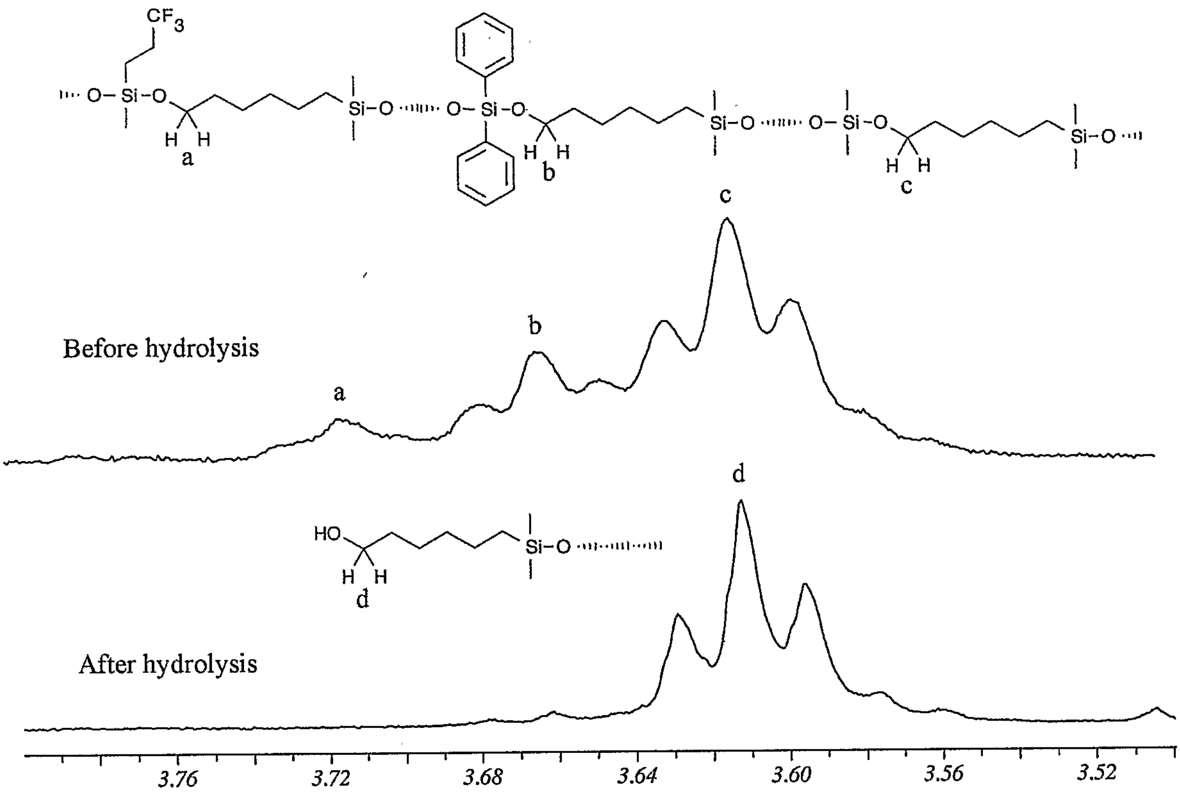
Before hydrolysis
After hydrolysis
3.76
3.72
3.68
3.64
3.60
3.56
3.52

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63786104 P
- US 04030938 A
- US 5278258 A
- US 5391590 A
- US 5411553 A
- US 5476515 A
- WO 0176651 A
- EP 629648 A
- US 5292849 A
- US 2007073031 A

### Non-patent literature cited in the description

- **HAEFLIGER et al.** *J. Refractive and Corneal Surgery,* 1994, vol. 10, 550-555
- **HETTLICH et al.** *J. Cataract Refract. Surg.,* 1994, vol. 20, 115-123
- **GROOT et al.** *J. Biomacromolecules,* 2003, vol. 4, 608-616
- **YILGOR et al.** *Polym. Bull.,* 1998, vol. 40, 525-532
- **KOJIMA et al.** *J. Polym. Sci., A-1,* 1966, (4), 2325-2327
- **HARRIS et al.** Reactive Oligomers, ACS Symposium Series. ACS, 1985, vol. 282, 161-174
- **MERKER et al.** *J. Polym. Sci.,* 1960, vol. 43, 297-310